# EUROPEAN PATENT SPECIFICATION

(11) **EP 1 819 290 B1**
(45) Date of publication and mention of the grant of the patent: **02.03.2011**
(21) Application number: 05770736.6
(22) Date of filing: 08.07.2005
(51) Int. Cl.: C12N 5/077, A61B 19/00, A61L 27/38, A61L 27/52, A61F 2/30

(54) **SCAFFOLDLESS CONSTRUCTS FOR TISSUE ENGINEERING OF ARTICULAR CARTILAGE**
GERÜSTLOSE KONSTRUKTE FÜR DIE ERZEUGUNG VON GELENKKNORPEL AUS GEWEBE
CONSTRUCTIONS SANS ECHAFAUDAGE POUR GENIE TISSULAIRE DE CARTILAGE ARTICULAIRE

(30) Priority: 09.07.2004 US 586862 P
(43) Date of publication of application: 22.08.2007
(73) Proprietor: WILLIAM MARSH RICE UNIVERSITY, Houston, TX 77005 (US)
(72) Inventor: Athanasiou, Kyriacos, A., 6100 Main Street, Houston, TX 77005 (US); Hu, Jerry, C., 6100 Main Street, Houston, TX 77005 (US)
(74) Representative: Pilkington, Stephanie Joan
(86) International application number: PCT/US2005/024269
(87) International publication number: WO 2006/017176

(56) References cited:
- WO-A1-97/17038
- WO-A2-02/10348
- TACCHETTI C ET AL: "In vitro morphogenesis of chick embryo hypertrophic cartilage." THE JOURNAL OF CELL BIOLOGY, vol. 105, no. 2, 1 August 1987 (1987-08-01), pages 999-1006, XP002485820 ISSN: 0021-9525
- TURNAY J ET AL: "Changes in the expression of annexin A5 gene during in vitro chondrocyte differentiation: influence of cell attachment." JOURNAL OF CELLULAR BIOCHEMISTRY, vol. 84, no. 1, 2002, pages 132-142, XP002971303 ISSN: 0730-2312
- SABBATINI A ET AL: "Use of human chondrocyte cell cultures to identify and characterize reactive antibodies in rheumatoid arthritis sera." CLINICAL AND EXPERIMENTAL RHEUMATOLOGY, vol. 21, no. 5, September 2003 (2003-09), pages 587-592, XP008092656 ISSN: 0392-856X
- LEBARON R G & ATHANASIOU K A: "Ex vivo synthesis of articular cartilage." BIOMATERIALS, vol. 21, no. 24, December 2000 (2000-12), pages 2575-2587, XP004217421 ISSN: 0142-9612
- MAUCK R L ET AL: "The role of cell seeding density and nutrient supply for articular cartilage tissue engineering with deformational loading." OSTEOARTHRITIS AND CARTILAGE, vol. 11, no. 12, December 2003 (2003-12), pages 879-890, XP002485821 ISSN: 1063-4584
- HU J C ET AL: "A self-assembling process in articular cartilage tissue engineering." TISSUE ENGINEERING, vol. 12, no. 4, April 2006 (2006-04), pages 969-979, XP002485822 ISSN: 1076-3279
- MASUDA K ET AL: 'A novel two-step method for the formation of tissue-engineered cartilage by mature bovine chondrocytes: the alginate-recovered-chondrocyte (ARC) method.' J ORTHOP RES. vol. 21, 2003, pages 139 - 148, XP002995911
- KOKENYESI R ET AL: 'Proteoglycan Production by Immortalized Human Chondrocyte Cell Lines Cultured under Conditions That Promote Expression of the Differentiated Phenotype.' ARCH BIOCHEM BIOPHYS. vol. 383, no. 1, 01 November 2000, pages 79 - 90, XP002995912
- DESSAU W ET AL: 'Extracellular Matrix Formation by Chondrocytes in Monolayer Culture.' J CELL BIOL. vol. 90, July 1981, pages 78 - 83, XP002995913
- CASTAGNOLA P ET AL: "Type X collagen synthesis during in vitro development of chick embryo tibial chondrocytes." THE JOURNAL OF CELL BIOLOGY, vol. 102, no. 6, June 1986 (1986-06), pages 2310-2317, ISSN: 0021-9525

## Description

The present application relates to tissue engineering. Tissue engineering is an area of intense effort today in the field of biomedical sciences. In vitro cell attachment, spreading and replication have been demonstrated to occur on various substrates, and the formation of solid tissue masses has been demonstrated for tissues such as cartilage. Most methods for preparing cartilaginous constructs are directed towards the use of various scaffolds as cell carriers. Typically, the seeded chondrocytes migrate from the scaffold to the bottom of the tissue culture vessel or well, even if the plates are not treated to promote cell adhesion. Typically, cells plated on non-tissue-treated plates will still attach eventually. Within a week of culture, proteins made by the chondrocytes or supplied in the medium have usually adsorbed onto the bottom of the wells to promote attachment. This results in a reduction in the size of the construct. Another drawback is that the attached cells tend to flatten and change to a different phenotype. Those cells compete with the remaining chondrocytes for nutrients and do not produce the desired extracellular matrix proteins for cartilage regeneration.

Certain culture techniques have been investigated for producing cartilage, including pellet culture^{1,2,3} and aggregate culture.³ In "pellet culture," isolated chondrocytes were first centrifuged into pellets. After a couple of days of culture in the centrifuge tubes (to allow the mass of cells to aggregate), these pellets were then transferred onto various surfaces, including hydrogels, or left in the centrifuge tubes for culture. In "aggregate culture," a low density of cells per surface area were maintained in hydrogel-coated six-well plates with or without gentle swirling. A cell suspension cultured in these hydrogel-coated plates form aggregates within the first 72 h of culture.³ The resultant aggregates were then cultured in hydrogel coated wells. Other culture techniques involving agarose include cell encapsulation,⁴ in which chondrocytes are mixed into molten agarose to result in a construct the contains agarose throughout. There remains a need for a suitable way to produce cartilage tissue constructs that maintain the appearance and characteristics of normal chondrocytes.

Cultures of chicken chondrocytes are disclosed by:
TACCHETTI C ET AL: "In vitro morphogenesis of chick embryo hypertrophic cartilage."THE JOURNAL OF CELL BIOLOGY, vol. 105, no. 2. 1 August 1987 (1987-08-01), pages 999-1006, ISSN: 0021-9525; and
TURNEY J ET AL: "Changes in the expression of annexin A5 gene during in vitro chondrocyte differentiation: influence of cell attachment." JOURNAL OF CELLULAR BIOCHEMISTRY, vol. 84, no. 1, 2002, pages 132-142, ISSN: 0730-2312.

Human chondrocytes are disclosed by:
SABBATINI A ET AL: "Use of human chondrocyte cell cultures to identify and characterize reactive antibodies in rheumatoid arthritis sera." CLINICAL AND EXPERIMENTAL RHEUMATOLOGY, vol. 21, no. 5, September 2003 (2003-09), pages 587-592, ISSN: 0392-856X.

The present invention seeks to overcome some of the drawbacks inherent in the prior art by providing methods for use in culturing chondrocytes to form constructs which contain higher percentages of cells that retain the chondrocytic phenotype. Another aspect of the present invention relates to tissue engineered constructs useful for forming neo-cartilage containing compositions for a variety of in vitro and in vivo purposes. Still another aspect of the invention relates to methods of treating individuals in need of articular cartilage growth. These and other objects can be achieved by the process as defined in the independent claims. Further enhancements are defined in the dependent claims.

In accordance with certain embodiments of the present invention, a process of producing a tissue-engineered articular cartilage construct is provided which comprises: a) coating at least one surface of a tissue culture vessel with a suitable material that is not conducive to cell attachment (e.g., a hydrogel); b) introducing onto each material-coated surface a suspension of living chondrocytes in culture medium wherein the suspension comprises about 1 x 10⁶ or more live chondrocytes per cm² of the at least one material-coated surface; c) allowing the chondrocytes to sediment on the coating material to form a cell aggregate. This process of cell aggregation to result in a construct, or an intermediate thereof, is termed the "Self-Assembling Process." The process further includes d) culturing the aggregate to yield the cartilage construct, or intermediate thereof. For ease of reference, the steps of the process are denoted as steps a, b, c, and so forth. A fixed order of performance of steps is not necessarily implied by the order in which the steps are listed, however. In some embodiments, step a) includes coating one or more surface of the culture vessel with the hydrogel. Alternatively, the culture vessel (e.g., a plurality of wells) is fashioned out of a hydrogel mold, or a hydrogel is used to form one or more cell culture well or vessel. In some embodiments, step d) yields the intermediate, and the process also includes e) seeding the intermediate with additional living chondrocytes; and f) culturing the seeded intermediate to enhance the thickness of the construct or intermediate thereof In preferred embodiments, step b) of an above-described process includes seeding each hydrogel-coated surface with a suspension containing at least 25 x 10⁶ live chondrocytes per cm² of hydrogel-coated surface. In certain embodiments, an above-described process comprises selecting a hydrogel comprising agarose, alginate or polyHEMA. In some embodiments the comprises 0.5 - 4 % (w/v) agarose, preferably 2%.

Also provided in accordance with certain embodiments of the present disclosure is a composition comprising at least one tissue-engineered scaffoldless construct prepared by a process as described above, and comprising a multiplicity of rounded, differentiated living chondrocytes. some embodiments, the construct comprises a periphery that is substantially devoid of non-phenotypic chondrocytes. In preferred embodiments the construct comprises chondrocytes capable of producing collagen type II. Some embodiments provide a composition wherein the construct comprises a compression modulus at least one-fourth as great as that of native bovine elbow cartilage. In some embodiments, the compression modulus of the construct is at least one-third that of native bovine elbow cartilage. Another composition provided by the present disclosure comprises a biphasic plug including a bone component and an above-described tissue-engineered cartilage construct.

Disclosed is a method of treating an individual in need of articular cartilage replacement is provided. This method comprises implanting at a site in the individual where articular cartilage is desired a composition comprising at least one tissue-engineered construct containing a multiplicity of rounded, differentiated living chondrocytes prepared by a process as described above.

The present disclosure also provides a method of treating an individual in need of articular cartilage replacement comprising implanting at a site in the individual where articular cartilage is desired a composition comprising at least one tissue-engineered construct comprising a composition as described above.

These and other embodiments, features and advantages of the present invention will become apparent with reference, by way of example, to the following description and drawings.
Fig. 1 is a photomicrograph showing a top view of disks from a 12 mm bowl-shaped cartilage construct prepared according to an embodiment of the present invention;
Fig. 2 is a photomicrograph showing a side view of the disks in Fig. 1. Each mark represents 1 mm;
Fig. 3 is a photomicrograph of 14 ttm sections of safranin-O/fast green stained disks, similar to those shown in Fig. 1;
Fig. 4 is a photomicrograph of 14 pm sections stained for collagen type II, similar to that shown in Fig. 1;
Fig. 5 is a graph showing the correlation of aggregate modulus (H_{A}) values of native articular cartilage and constructs formed over agarose to GAG/DW and to collagen/DW. HA shows a strong positive correlation with collagen/DW (R² = 1.00) and a strong negative correlation with GAG/DW (R² = 0.99);

A new process of making a construct comprising chondrocytes generally includes taking isolated chondrocytes in suspension, allowing the cells to sediment onto one or more coated tissue culture surface, in which the coating comprises a material that is not conducive to cell attachment and which is non-toxic and otherwise suitable for inclusion in a tissue culture environment. The sedimenting cells aggregate and grow into constructs that contain rounded chondrocytes and which contain collagen and glycosaminoglycan throughout. As used herein, a "construct" or "tissue-engineered construct" refers to a three-dimensional mass having length, width and thickness, and which comprises living mammalian tissue produced in vitro. The process of cells aggregating to result in a construct is termed the "Self-Assembling Process." The new constructs also demonstrate mechanical properties, such as compression modulus, which improve over time in culture. Thus, the process described herein forms articular cartilage in vitro without the use of scaffolds. The following description is offered by way of illustration.

### Chondrocyte Isolation and Seeding.

Articular chondrocytes were isolated from the distal femur of week old male calves (Research 87 Inc., Boston, MA), less than 36 hours after slaughter, with collagenase type I (Worthington, NJ) in culture medium. The medium was DMEM with 4.5 g/L-glucose and Lglutamine (Biowhittaker), 10% fetal bovine serum (Biowhittaker), fungizone (Biowhittaker), penicillin/streptomycin (Biowhittaker), non-essential amino acids (Life Technologies), 0.4 mM proline (ACS chemicals), 10 mM HEPES (Fisher Scientific), and 50 µg/ml L-ascorbic acid (Acros Organics). Chondrocytes were frozen in culture medium supplemented with 20% FBS and 10% DMSO at -80°C for 2 weeks to a month before cells from two donor legs were pooled together. Alternatively, fresh cells are used. Due to the joint capsule, articular cartilage exists in an "immune privileged" state. For this reason, xenogenic articular cartilages (i.e., cartilage produced from bovine or porcine cells) are viable options for implantation in many instances. Alternatively, if the construct is to be used for in vivo tissue replacement, the source of articular chondrocytes may be autologous cartilage from a small biopsy of the patient's own tissue, provided that the patient has healthy articular cartilage that may be used as the start of in vitro expansion. Another suitable source of chondrocytes is heterologous chondrocytes from histocompatible cartilage tissue obtained from a donor or cell line.

### Hydrogel Coating of Well Plates.

The bottoms and sides of 96-well plates were coated with 100 µA12% agarose (w/v), and the plates were shaken vigorously to remove excess agarose. The surface area at the bottom of well in a 96-well plate is 0.2 cm². Chilled plates were then rinsed with culture medium before the introduction of cells. While 2% agarose is a preferred concentration, acceptable results may be obtained any agarose concentration in the range of about 0.5% to about 4%. The use of lower concentrations of agarose offer the advantage of reduced costs; however, at concentrations below about 1% the agarose does not stiffen enough for optimal ease of handling. An alternative to well plates is wells made completely of agarose.

As an alternative to agarose, another type of suitable hydrogel (e.g., alginate and/or 30 PolyHEMA) may be used. A "hydrogel" is a colloid in which the particles are in the external or dispersion phase and water is in the internal or dispersed phase. Suitable hydrogels are non-toxic to the cells, do not induce chondrocyte attachment, allow for the diffusion of nutrients, do not degrade significantly during culture, and are firm enough to be handled. The results obtained using agarose are considered to be representative of results that will be obtained with other suitable hydrogels.

### Chondrocyte Sedimentation.

Articular chondrocytes were thawed in suspension. This suspension was then introduced into the hydrogel-coated wells at 5 x 10⁶ cells per well in 300 µl of culture medium (5 x 10⁶ cells/0.2 cm² hydrogel-coated surface). The chondrocytes sedimented and formed a continuous cell layer within 24 hours, from which time 200 µl of the medium was changed daily. After 1 month of culture, these chondrocyte constructs were transferred to hydrogel-coated 48-well plates, with 1 ml culture medium. The hydrogel-coated culture area of each well in the 48-well plate is 0.95 cm2. From that point on, 800 µl of culture medium was changed daily. Time zero is defined as the day the chondrocytes were seeded.

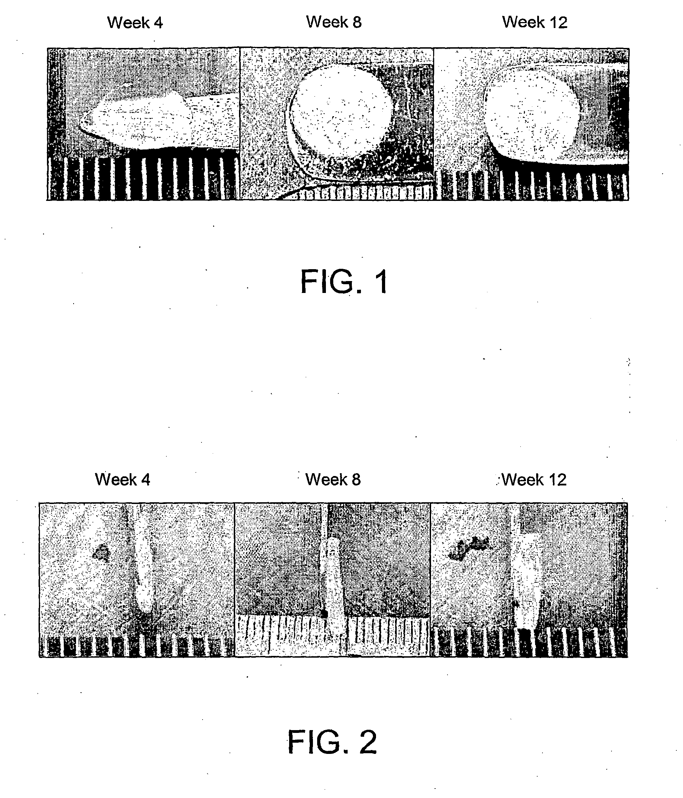

The cell suspension was directly introduced onto hydrogel-coated wells. The chondrocytes underwent a Self-Assembling Process and were cultured in these wells. Chondrocytes sedimented into an aggregate within 24 hours after seeding. At t =4 weeks, microscopic examination revealed that the cells were still round within the engineered constructs, indicating that the chondrocytic phenotype was maintained. The constructs showed a slight curl all around the edges, like a bowl, and measured roughly 8 mm in diameter when flattened. The thickness of the constructs at that time was about 0.5 mm. By t = 7 weeks, the constructs had grown to more than 10 mm in diameter when flattened. The thickness of the construct is approximately 1.0 mm. Fig. 1 shows a 6 mm diameter by 1 mm thick disc punched out from a 12 mm bowl shaped construct. Fig. 2 shows the same disc viewed from the side. In the photographs, each mark represents 1 mm.

### Histological Evaluation.

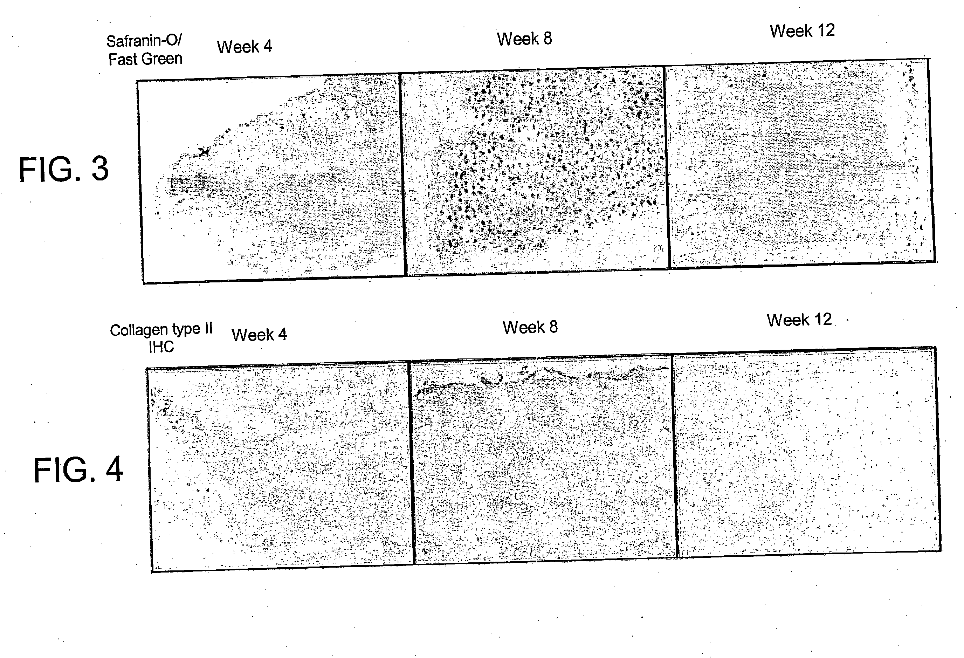

Histological evaluation of a safranin-O/fast green stained 14 µm section of the disc showed glycosaminoglycan (Fig. 3) and a 14 µm collagen type II IHC stained section of the disc revealed collagen type II (Fig. 4) throughout the engineered construct. These observations suggest that the chondrocyte cells of the construct maintain their phenotypic functions. Notably, the extracellular matrix produced in vitro by spherical chondrocytes have been shown to comprise collagen type II. By contrast, it has also been shown that the flattened, non-phenotypic chondrocytes produce collagen type I. Since collagen type II is the predominant collagen type in cartilage, in vitro culturing of chondrocytes may include stimulating the cells to produce collagen type II.

### Mechanical Property.

"Aggregate modulus" is a conventional measurement used in characterizing cartilage. Suitable measuring devices are known in the art.^{5,6} In early studies, mechanical testing of the representative aggregate or construct yielded an aggregate modulus of 4 kPa at 4 weeks after seeding, increasing to approximately 50 kPa at 7 weeks from initial seeding of the culture. These results are quite significant, for they suggest that the engineered constructs at 7 weeks have considerable structural integrity. For instance, this aggregate modulus at 7 weeks is approximately one-fourth that of native bovine elbow cartilage (about 200 kPa). Further studies yielded mechanical data presented in Table 1.

**TABLE 1**

| **Mechanical Properties of Constructs Cultured over the Agarose Substratum and on Tissue Cultured Plastic (TCP)*** | | | |
|---|---|---|---|
| | HA (kPa) | K (10⁻¹⁰m⁴Ns) | v |
| Week 4, over agarose | 19± 3 | 17± 6 | 0.23±0.08 |
| Week 8, over agarose | 43±13 | 40±21 | 0.11±0.08 |
| Week 12, over agarose | 53±9 | 22±24 | 0.03±0.05 |
| Week 4, on TCP | 13±4 | 24±10 | 0.22±0.11 |
| Week 8, on TCP | 19±3 | 33±21 | 0.07±0.09 |
| Articular Cartilage | 139±41 | 42±28 | 0.01±0.01 |

| | | | |
|---|---|---|---|
| *Data are shown as mean ± standard deviation. | | | |

As shown by Table 1, constructs formed over agarose using the Self-Assembling Process have better mechanical properties than those formed over TCP.

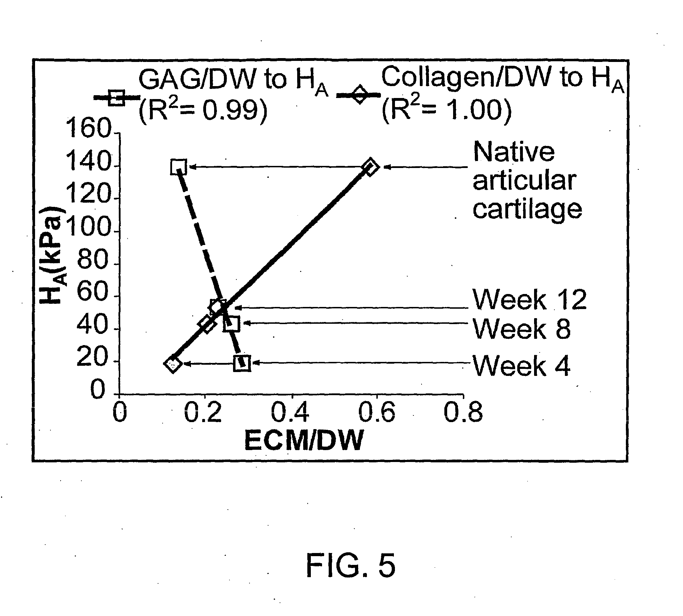

The constructs were observed to continue to increase in mechanical properties up to at least 12 weeks. In addition to increased mechanical properties, the biochemical content of the constructs also trended towards native tissue (Fig. 5). Fig. 5 shows that from week 4 to week 12, the correlations between construct mechanical properties (y-axis) and biochemical properties (x-axis) are linear relationships. Furthermore, the relationship between the mechanical and biochemical properties of native tissue falls on the same trend. Construct mechanical properties may eventually reach that of native tissue given longer culture periods or the application of biochemical/biomechanical stimuli.

The methods described herein avoid some of the undesirable consequences of cell attachment to a scaffold or other surface, in which the scaffold or surface is designed to promote cell attachment. Common disadvantages exhibited by previous cell culture methods are listed in Table 2. In the present case, attachment is not desirable since the most efficient use of chondrocytes employs the largest percentage of rounded cells as possible. In the representative example above, a cell suspension is directly introduced into hydrogel-coated wells. The chondrocytes pack slowly into an aggregate and are cultured in these wells. By coating the wells with a hydrogel, the chondrocytes remain round and differentiated. Nutrients are able to diffuse into the bottom of the constructs from the hydrogel, in contrast to cultures in which the construct is in contact with a plastic surface. The resulting tissue constructs will be advantageously employed for tissue replacement, as well as for use as tissue substitutes for cell culture and in construction of prostheses. Unlike the tissue engineering methods that employ scaffolds, a thick (e.g., tens of microns) capsule of flattened cells does not form around the present scaffoldless constructs. Certain advantages of the present self-assembling process are listed in Table 2 and are contrasted with common drawbacks of conventional processes.

**TABLE 2**

| Comparison of Scaffold Use to Self-Assembling Process | | |
|---|---|---|
| | Possible Scaffold Problems | Self assembling Solutions |
| 1. | | No extra seeding stress. |
| | Stressful seeding processes such as cross-linking process (toxic polymerization activators or UV) and spinner flask shear. | |
| 2. | Loss of phenotype associated with some solid scaffolds. | Phenotype shown to be retained in self-assembling process. |
| 3. | Inhibition of cell migration and cell to cell communication. | Cells are initially in direct contact with each other. |
| 4. | Stress shielding of cells from mechanotransduction. | The entirety of neotissue is exposed to mechanical stimuli. |
| 5. | Scaffold obstructs cell growth and ECM remodeling. | Unobstructed ECM production/ remodeling. |
| 6. | Toxic degradation products. | No degradation products. |
| 7. | Inflammatory response towards scaffold. | No scaffold for body to react to. |
| 8. | Invasion of other cell types into scaffold. | Cells form cohesive construct that has no space for other cells. |

To restore function to an articular defect it is best to avoid introducing additional problems, as often occurs with existing scaffold-based constructs (Table 2). Other techniques which rely on the use of chondrocytes encapsulated in agarose⁴ may eventually encounter problems such as the persistence of the biomaterial or matrix remodeling being hindered by the biomaterial. In contrast, the present methods employ as an external support a layer or substratum of a material that is not conducive to cell adhesion to the coated surface. Thus, the surface coating material or support layer is easily removed from the cultured construct (e.g., by peeling away the hydrogel layer from the finished construct). Other procedures, utilizing agarose encapsulation, suffer from a disadvantage when attempting to free the construct from the agarose. This problem occurs due to the fact that chondrocytes, and thus the tissue formed, are encapsulated in the agarose. As a consequence, agarose is well integrated into the resulting construct such that the agarose can no longer be removed. Such constructs, containing embedded agarose, would not be expected to be satisfactory for in vivo implantation.

In the foregoing examples, chondrocytes were cultured in 96-well plates. However, the chondrocytes will behave similarly regardless of the well size. The hydrogel coating and a sufficient cell density for seeding being the most important factors. Depending on the size of the cells (i.e., cells from different species, zones, and passage numbers may vary in size), fewer than roughly 1 million cells per cm² of hydrogel surface will fail to cover the entire surface area with at least one layer of cells, and therefore will tend to result in aggregates that are not continuous. Thus, when too few cells are seeded, the cells do not form a continuous sheet of cartilage. Seeding more than the above-described 25 x 10⁶ chondrocytes per cm² hydrogel-coated surface can be used to produce constructs that are thicker. To produce a prosthesis or a tissue substitute for cell culture, the above-described process may be scaled up simply by coating Petri-dishes with hydrogels and seeding the appropriate number of cells. Plugs can then be punched out from the sheet of neo-tissue that will form. A 10 cm diameter Petri-dish will yield 78.5 cm² of neo-tissue, enough to re-surface about half of an adult knee, which ranges from 10²-10³ cm².⁽⁷⁾

Constructs may also be engineered with a "bone" component to result in a biphasic plug that will be easily transplanted into diseased areas. Tissue-engineered constructs prepared as described herein may be used in prosthetic devices for the repair or replacement of damaged cartilage, such as articular joint cartilage. The techniques used for implanting the formed cartilaginous constructs will be similar to those now used for arthroplasty procedures. Cartilaginous constructs prepared as described herein may also find use as a tissue substitute for cell culture.

Without further elaboration, it is believed that one skilled in the art can, using the description herein, utilize the present invention to its fullest extent. The foregoing embodiments are to be construed as illustrative, and not as constraining any of the disclosure.

### References

1. Graff RD, Kelley SS, Lee GM. Role of pericellular matrix in development of a mechanically functional neocartilage. Biotechnol Bioeng, 2003 May 20; 82(4): 457-64.
2. Malda J, Kreijveld E, Temenoff JS, et al. Expansion of human nasal chondrocytes on 30 macroporous microcarriers enhances redifferentiation. Biomaterials, 2003 Dec; 24(28): 5153-61.
3. Stewart MC, Saunders KM, Burton-Wurster N, and Macleod IN. Phenotypic stability of articular chondrocytes in vitro: The effects of culture models, bone morphogenetic protein 2, and serum supplementation. Journal of Bone and Mineral Research, 2000 Jan; 15(1): 166-7.
4. Mauck, RL, et al., The role of cell seeding density and nutrient supply for articular cartilage tissue engineering with deformational loading. Osteoarthritis Cartilage, 2003. 11(12): p. 879-90.
5. Athanasiou, KA, Agarwal, A, and Dzida, FJ, Comparative study of the intrinsic mechanical properties of the human acetabular and femoral head cartilage. J Orthop Res, 1994. 12(3): p. 340-9.
6. Mow, VC, et al., Biphasic creep and stress relaxation of articular cartilage in compression. Theory and experiments. J Biomech Eng, 1980.102(1): p. 73-84.
7. Eckstein F, Winzheimer M, Hohe J, et al.. Interindividual variability and correlation among morphological parameters of knee joint cartilage plates: analysis with three-dimensional MR imaging. Osteoarthritis and Cartilage, 2001 Feb; 9(2): 101-11.

The present tissue engineered constructs containing living chondrocytes may be used for a variety of purposes both in vivo and in vitro. In addition to using the tissue-engineered constructs as prosthetic devices for the repair or replacement of damaged cartilage, such as articular joint cartilage tissue-engineered constructs, they can also serve as in vivo delivery systems for proteins or other molecules secreted by the cells of the construct. Still another use of tissue engineered constructs as an in vitro model of tissue function or as a model system for testing the effects of a treatment or drug of interest. It is also expected that the above-described hydrogel culturing methods will also be applicable to cell types other than articular chondrocytes.

## Claims

1. A process of producing a tissue-engineered articular cartilage construct comprising:
a) coating at least one surface of a tissue culture vessel with a coating material that is not conducive to cellular attachment;
b) introducing onto said at least one material-coated surface a suspension of live chondrocytes in culture medium, wherein the suspension comprises 1 x 10⁶ or more live chondrocytes per cm² of the at least one material-coated surface;
c) allowing the chondrocytes to sediment on said coating to form an initial cell aggregate through a self-assembling process; and
d) culturing said initial aggregate to yield a scaffoldless cartilage construct, or an intermediate thereof.

2. A process of producing a tissue-engineered articular cartilage construct comprising:
a) forming a plurality of tissue culture wells from a polymeric material that is not conducive to cellular attachment;
b) introducing onto said wells a suspension of live chondrocytes in culture medium, wherein the suspension comprises 1 x 10⁶ or more live chondrocytes per cm² of the well surface;
c) allowing the chondrocytes to sediment in said wells to form an initial cell aggregate through a self-assembling process; and
d) culturing said initial aggregate to yield a scaffoldless cartilage construct, or an intermediate thereof.

3. The process of claim 1 or 2, wherein step d) yields said intermediate, and said process comprises
e) seeding said intermediate with additional living chondrocytes; and
f) culturing said seeded intermediate to enhance the thickness of said construct or intermediate thereof.

4. The process according to one of the preceding claims, wherein step b) comprises seeding each said material-coated surface with a suspension comprising at least 25 x 10⁶ live chondrocytes per cm² of material-coated area.

5. The process according to one of the preceding claims, wherein said material comprises a hydrogel.

6. The process of claim 5 wherein said hydrogel is chosen from the group consisting of agarose, alginate and polyHEMA.

7. The process of claim 6 wherein said hydrogel comprises 0.5 - 4 % (w/v) agarose.

8. The process of claim 7 wherein said hydrogel comprises 2% (w/v) agarose.

9. The method of any one of claims 1 to 8 further comprising the step of forming a biphasic plug comprising a bone component and the scaffoldless cartilage construct.

10. The method of any one of claims 1 to 8 further comprising the step of using the scaffoldless cartilage construct in forming a prosthetic device for the repair or replacement of damaged cartilage.

11. The method of any one of claims 1 to 8 further comprising the step of punching plugs from the scaffoldless cartilage construct.

## Patentansprüche

1. Verfahren zur Herstellung eines Gelenkknorpelkonstrukts durch Gewebezüchtung (Tissue-Engineering), Folgendes umfassend:
a) Beschichten mindestens einer Oberfläche eines Gewebekulturgefäßes mit einem Beschichtungsmaterial, das die Zellanhaftung nicht fördert,
b) Zuführen einer Suspension aus lebenden Knorpelzellen in Kulturmedium auf die mindestens eine materialbeschichtete Oberfläche, wobei die Suspension 1 x 10⁶ oder mehr lebende Knorpelzellen pro cm² der mindestens einen materialbeschichteten Oberfläche umfasst,
c) Ermöglichen, dass sich die Knorpelzellen auf der Beschichtung ablagern, um durch einen Selbstansammlungsprozess ein anfängliches Zellaggregat zu bilden, und
d) Kultivieren des anfänglichen Aggregats, um ein gerüstloses Knorpelkonstrukt oder eine Zwischenstufe davon zu erzielen.

2. Verfahren zur Herstellung eines Gelenkknorpelkonstrukts durch Gewebezüchtung (Tissue Engineering), Folgendes umfassend:
a) Bilden mehrerer Vertiefungen (Wells) für Gewebekulturen aus einem Polymermaterial, das die Zellanhaftung nicht fördert,
b) Zuführen einer Suspension aus lebenden Knorpelzellen in Kulturmedium auf die Vertiefungen, wobei die Suspension 1 x 10⁶ oder mehr lebende Knorpelzellen pro cm² der Oberfläche der Vertiefung umfasst,
c) Ermöglichen, dass sich die Knorpelzellen in den Vertiefungen ablagern, um durch einen Selbstansammlungsprozess ein anfängliches Zellaggregat zu bilden, und
d) Kultivieren des anfänglichen Aggregats, um ein gerüstloses Knorpelkonstrukt oder eine Zwischenstufe davon zu erzielen.

3. Verfahren nach Anspruch 1 oder 2, wobei Schritt d) eine Zwischenstufe erzielt und das Verfahren Folgendes umfasst:
e) Impfen der Zwischenstufe mit zusätzlichen lebenden Knorpelzellen und
f) Kultivieren der geimpften Zwischenstufe, um die Dicke des Konstrukts oder der Zwischenstufe davon zu erhöhen.

4. Verfahren nach einem der vorhergehenden Ansprüche, wobei Schritt b) das Impfen jeder materialbeschichteten Oberfläche mit einer Suspension umfasst, die mindestens 25 x 10⁶ lebende Knorpelzellen pro cm² des materialbeschichteten Bereichs umfasst.

5. Verfahren nach einem der vorhergehenden Ansprüche, wobei das Material ein Hydrogel umfasst.

6. Verfahren nach Anspruch 5, wobei das Hydrogel aus der Gruppe ausgewählt ist, die aus Agarose, Alginat und Poly-HEMA besteht.

7. Verfahren nach Anspruch 6, wobei das Hydrogel 0,5 bis 4 % Gewichtsvolumen (w/v) Agarose umfasst.

8. Verfahren nach Anspruch 7, wobei das Hydrogel 2 % Gewichtsvolumen (w/v) Agarose umfasst.

9. Verfahren nach einem der Ansprüche 1 bis 8, ferner den Schritt des Bildens eines Zwei-Phasen-Implantats umfassend, das eine Knochenkomponente und das gerüstlose Knorpelkonstrukt umfasst.

10. Verfahren nach einem der Ansprüche 1 bis 8, ferner den Schritt des Verwendens des gerüstlosen Knorpelkonstrukts beim Bilden einer Prothesevorrichtung zur Wiederherstellung oder zum Austausch beschädigten Knorpels umfassend.

11. Verfahren nach einem der Ansprüche 1 bis 8, ferner den Schritt des Stanzens von Implantaten aus dem gerüstlosen Knorpelkonstrukt umfassend.

## Revendications

1. Procédé de fabrication d'une construction de cartilage articulaire par génie tissulaire, comprenant :
a) le revêtement d'au moins une surface d'un flacon à culture tissulaire avec un matériau de revêtement qui ne favorise pas l'attachement cellulaire ;
b) l'application sur ladite au moins une surface revêtue du matériau d'une suspension de chondrocytes vivants dans un milieu de culture, dans laquelle la suspension comprend 1 x 10⁶ chondrocytes vivants ou plus par cm² de ladite au moins une surface revêtue du matériau ;
c) le fait de laisser les chondrocytes sédimenter sur ledit revêtement pour former un agrégat cellulaire initial par un procédé d'auto-assemblage ; et
d) la culture dudit agrégat initial pour donner une construction de cartilage sans échafaudage, ou un produit intermédiaire de celle-ci.

2. Procédé de fabrication d'une construction de cartilage articulaire par génie tissulaire, comprenant :
a) la formation d'une pluralité de puits de culture tissulaire à partir d'un matériau polymère qui ne favorise pas l'attachement cellulaire ;
b) l'application sur lesdites puits d'une suspension de chondrocytes vivants dans un milieu de culture, dans laquelle la suspension comprend 1 x 10⁶ chondrocytes vivants ou plus par cm² de surface de puits ;
c) le fait de laisser les chondrocytes sédimenter dans lesdits puits pour former un agrégat cellulaire initial par un procédé d'auto-assemblage ; et
d) la culture dudit agrégat initial pour donner une construction de cartilage sans échafaudage, ou un produit intermédiaire de celle-ci.

3. Procédé selon la revendication 1 ou 2, dans lequel l'étape d) donne ledit produit intermédiaire, et ledit procédé comprend
e) l'ensemencement dudit produit intermédiaire avec des chondrocytes vivants supplémentaires ; et
f) la culture dudit produit intermédiaire ensemencé pour accroître l'épaisseur de ladite construction ou dudit produit intermédiaire de celle-ci.

4. Procédé selon l'une quelconque des revendications précédentes, dans lequel l'étape b) comprend l'ensemencement de chaque dite surface revêtue du matériau avec une suspension comprenant au moins 25 x 10⁶ chondrocytes vivants par cm² d'aire revêtue avec le matériau.

5. Procédé selon l'une quelconque des revendications précédentes, dans lequel ledit matériau comprend un hydrogel.

6. Procédé selon la revendication 5, dans lequel ledit hydrogel est choisi dans le groupe constitué par l'agarose, un alginate et un polyHEMA.

7. Procédé selon la revendication 6, dans lequel ledit hydrogel comprend de 0,5 à 4 % (p/v) d'agarose.

8. Procédé selon la revendication 7, dans lequel ledit hydrogel comprend 2 % (p/v) d'agarose.

9. Procédé selon l'une quelconque des revendications 1 à 8, comprenant en outre l'étape consistant à former un bouchon à deux phases comprenant un constituant osseux et la construction de cartilage sans échafaudage.

10. Procédé selon l'une quelconque des revendications 1 à 8, comprenant en outre l'étape consistant à utiliser la construction de cartilage sans échafaudage dans la formation d'un dispositif prothétique pour la réparation ou le remplacement de cartilage endommagé.

11. Procédé selon l'une quelconque des revendications 1 à 8, comprenant en outre l'étape consistant à perforer des bouchons à partir de la construction de cartilage sans échafaudage.
